# EUROPEAN PATENT APPLICATION

(11) **EP 4 714 397 A1**
(43) Date of publication of application: **25.03.2026**
(21) Application number: 24202351.3
(22) Date of filing: 24.09.2024
(51) Int. Cl.: A61F 2/07

(54) **AGE ADAPTABLE FONTAN TUNNEL**

(71) Applicant: Universiteit van Amsterdam, 1012 WX Amsterdam (NL)
(72) Inventor: ZÁVODSZKY, Gábor, 1012 WX Amsterdam (NL)
(74) Representative: V.O.

(57) **Abstract**

A conduit for fluidly connecting two vessels of a mammal body, the conduit comprising a scaffold providing a circumferential support to the conduit and an elastomeric material provided on the scaffold, configured to form a fluid impermeable circumferential wall of the conduit conforming to the dimensions of the scaffold, ranging from a proximal end of the conduit to a distal end of the conduit. The conduit is configured such that dimensions of the conduit are adaptable to adjustments in dimensions of the vessels, the adjustments in dimensions of the vessels being driven by somatic growth of the mammal body.

## Description

### TECHNICAL FIELD

The present invention is related to the field of treatments for a congenital heart disease.

### BACKGROUND

Patients born with a univentricular heart have the most severe congenital heart defect with near-universal death without intervention. The gold standard treatment, the Fontan procedure, is designed to reroute blood flow from the veins directly to the pulmonary arteries (PA), by placing a vascular graft bypassing the heart and thereby reducing the workload on the single functional ventricle.

While the Fontan procedure, has improved survival greatly, morbidity remains high including liver and kidney diseases, thromboembolic events and reduced exercise capacity. Due to the young age (2-4 years) of the patients when this procedure is carried out, the patient may outgrow the size of the Fontan tunnel during natural growth as the current grafts lack growth potential.

### SUMMARY

The current conduits for a Fontan procedure lack adaptivity or growth potential. Therefore, the Fontan conduit becomes undersized when the patient grows older, due to the inability of the inelastic conduit to match the dimensions of the vessels being driven by somatic growth of the mammal body.

Alternative solutions, other than a Fontan procedure, such as a graft replacement and tissue-engineered graft encounter severe shortcomings. Graft replacement requires an invasive reoperation, leading to increased risk of morbidity and mortality and tissue-engineered grafts suffer from graft stenosis and unpredictable tissue growth that hinders their translation to clinical practice for the near future. Therefore, a conduit configured to be adaptable to adjustments in dimensions of the vessels driven by somatic growth of the patient, thereby supporting the unique circulation of the patient from child- to adulthood, is required.

A first aspect provides a conduit for fluidly connecting two vessels of a mammal body, the conduit comprising a scaffold providing a circumferential support to the conduit and an elastomeric material provided on the scaffold, configured to form a fluid impermeable circumferential wall of the conduit conforming to the dimensions of the scaffold, ranging from a proximal end of the conduit to a distal end of the conduit. The conduit is configured such that dimensions of the conduit are adaptable to adjustments in dimensions of the vessels, the adjustments in dimensions of the vessels being driven by somatic growth of the mammal body.

In an implementation of the conduit, the scaffold comprises, or is made of, auxetic structures. The auxetic structures of the scaffold may be on macroscopic level or microscopic level or a combination of both. The scaffold of the conduit may comprise, or be made of, the different types of auxetic structures belonging to the family of auxetic structures. Auxetic structures provide improved mechanical properties.

In another implementation of the conduit, the scaffold comprises, or is made of, auxetic structures and non-auxetic structures. This provides the scaffold with different mechanical properties.

In a further implementation, the scaffold is configured to provide circumferential structural support from a proximal end of the conduit to a distal end of conduit. In this way, the scaffold provides circumferential structural support over the entirety of the conduit, enhancing the mechanical properties of the conduit.

In yet another implementation, the scaffold is configured to provide circumferential structural support over partial segments of the conduit. In this way, a plurality of partial segments of the conduit is provided with structural support from the scaffold and a plurality of partial segments of the conduit is not provided with circumferential structural support from the scaffold. The plurality of partial segments of the conduit provided with structural support from the scaffold exhibits stronger mechanical properties than the plurality of partial segments of the conduit which are not provided with circumferential structural support from the scaffold.

In another implementation, the scaffold has an open cell design, wherein the scaffold of the conduit is configured to provide circumferential structural support to the conduit, ranging from a proximal end of the conduit to a distal end of the conduit. This allows for structural support over plurality of partial segments of the conduit, allowing natural flexibility and bending to the conduit.

In an implementation, the elastomeric material and auxetic material are biocompatible. This allows for safe implantation of the material in a patient.

In another implementation, the auxetic material may comprise, or be made of, polymers and elastomers and or hydrogels and or metal and alloys and or carbon-based materials and or a combination of these materials. More specifically, the auxetic material may comprise, or be made of, Co-Cr, surgical steel, nitinol or any other material comprising auxetic properties. These materials comprise auxetic properties while these materials are already well-established in the medical field.

In yet another implementation, the elastomeric material is configured to be compatible with the auxetic properties of the scaffold. In this way the elastomeric material is configured to maintain conformance to the dimensions of the scaffold in case of adjustments in dimensions of the scaffold. The elastomeric material comprises, or is made of, a resilient material. In this way, the elastomeric material is configured to adapt to the adjustments in dimensions of the scaffold.

In an embodiment, the scaffold and elastomeric material are configured to form the conduit. The scaffold forms the structural support of the conduit providing circumferential support to the conduit. The elastomeric material is provided on and in between the scaffold by means of for example dip coating, wherein the scaffold is dipped into the elastomeric material in its liquid phase, and then is withdrawn from the elastomeric material. Subsequently, a layer of the elastomeric material is formed on and in between the scaffold, which is configured to form a fluid impermeable circumferential wall of the conduit conforming to the dimensions of the scaffold, ranging from a proximal end of the conduit to a distal end of the conduit.

In an implementation of the conduit, wherein the conduit comprises the scaffold and the elastomeric material, the conduit may have additional coating layers on the surface of the elastomeric material, by for example means of surface integration coatings, barrier coatings or any other type of a coating layer. In this way, the surface of the conduit is modified such that it may inhibit adhesion of undesired cells and promote adhesion of desired cells after implantation in the mammal body to promote effective integration of the conduit in the mammal body. Another advantage of adding additional coating layers, is that the mechanical properties of the surface of the conduit may be modified such to obtain the required and or desired mechanical properties.

In another implementation, the conduit, and the scaffold in particular, is configured to be expanded concomitantly in radial and axial directions. This allows the conduit to expand uniformly in both the radial and the axial direction. This prevents the conduit from the phenomena of dog boning and foreshortening of the conduit when expanded.

In yet another implementation, the conduit is configured to be expanded from first dimensions to first expanded dimensions. The dimension of the conduit comprises the length of the conduit, the inner diameter of the conduit, the outer diameter of the conduit, and the circumferential wall thickness of the conduit. The first dimensions of conduit are compatible with the dimensions of the vessels of the patient during implantation, wherein the dimensions of the vessels of the patient comprise the inner diameter of the vessels, the outer diameter of the vessels, and the circumferential wall thickness of the vessels. The first dimensions of the conduit may become incompatible with the dimensions of the vessels of the patient after the first somatic growth. Therefore, the conduit can be expanded to the first expanded dimensions to remain compatible with the dimensions of the vessels of the patient after the first somatic growth. This allows for increased transportation volume for transporting blood and/or other fluids.

In another implementation, the conduit is configured to be expanded from first expanded dimensions to next expanded dimensions. The first expanded dimensions of the conduit become incompatible with the dimensions of the vessels of the patient after the next somatic growth. Therefore, the conduit can be expanded to next expanded dimensions to remain compatible with the dimensions of the vessels of the patient after the next somatic growth. It is preferred that in the expanded dimensions and the next expanded dimensions, the dimensions of the scaffold are substantially stable. This may be a requirement for a selection of one or more materials for manufacturing of the scaffold.

In yet another implementation, the conduit is configured to be expanded multiple times to further increment the dimensions of the conduit. This allows the conduit to be adaptable to adjustments in the dimensions of the vessels, the adjustments in dimensions of the vessels being driven by a somatic growth of the mammalian body.

Again, in another implementation, the conduit is configured to remain in its expanded dimensions. After each deformation, such as expansion, the scaffold may plastically remain in the specific expanded dimensions and the scaffold may not return back to its previous dimensions. In this way, the dimensions of the conduit remain compatible with the dimensions of the vessels of the mammal body.

In another implementation, the conduit is configured to be further expanded from its expanded dimensions. In this way the conduit can be adaptable to the next adjustments in dimensions of the vessels, the adjustments in dimensions of the vessels being driven by the somatic growth of the mammalian body.

In an implementation, the conduit, and the scaffold in particular, is configured to be expanded by means of an external mechanical force. In an example, the external mechanical force is applied by means of balloon angioplasty. The external mechanical force may also be applied by means of other expansion tools and methods.

In another implementation, the conduit, and the scaffold in particular, may be configured to self-expand because of tension forces applied by the vessels between which the conduit is implanted. In another example, the conduit, and the scaffold in particular, may be configured to self-expand in response to other mechanisms and or changes in environmental parameters and or environmental parameters acting upon the conduit.

In one example, wherein the scaffold is circumferentially ranging from a proximal end of the conduit to a distal end of the conduit, the conduit may be configured to be expanded concomitantly in axial and radial directions from the proximal end of the conduit to the distal end of the conduit. In another example, wherein the conduit has an open cell design, the scaffold is circumferentially ranging from a proximal end of the conduit to a distal end of the conduit, the open cell design conduit is configured to allow segment-by-segment expansion of the conduit. In this way, the expansion of the open cell conduit is carried out in a more controlled manner, enhancing patient safety.

In another example, the conduit may be configured to connect or reconnect two or more vessels of a mammal body. In yet another example, the conduit may be configured to replace a portion of a vessel of a mammal body.

In an implementation, the conduit is configured to form a fluid impermeable layer between an intravascular environment of the mammal body and an extravascular environment of the mammal body. In this way the conduit forms a fluid pathway wherein the intravascular environment of the mammal body is isolated from the extravascular environment of the mammal body and wherein the conduit is configured to facilitate the transportation of bodily fluids.

In yet another implementation, the conduit is patient specific, wherein the conduit is designed such that the dimensions of the conduit are compatible with the dimensions of the vessels between which the conduit is implanted. The conduit is configured such that expansion ratios of the conduit are pre-defined per patient. Meaning that, the dimensions of the conduit are adaptable to adjustments in dimensions of the vessels of the specific patient, the adjustments in dimensions of the vessels being driven by the somatic growth of the specific patient. This allows the continuous optimal compatibility of the conduit to the vessels of the patient.

### BRIEF DESCRIPTION OF THE DRAWINGS

The various aspects and examples thereof will now be discussed in conjunction with drawings. In the drawings:
Figure 1A: shows a conduit;
Figure 1B: shows a scaffold;
Figure 1C: shows an open cell conduit;
Figure 1D: shows a conduit between two vessels of a mammal body;
Figure 2A: shows a conduit in a not expanded dimension;
Figure 2B: shows a conduit with an uninflated balloon positioned within the internal environment of the conduit;
Figure 2C: shows a conduit with an inflated balloon positioned within the internal environment of the conduit;
Figure 2D: shows a conduit in an expanded dimension; and
Figure 2E: shows a conduit in a next expanded dimension.

### DETAILED DESCRIPTION

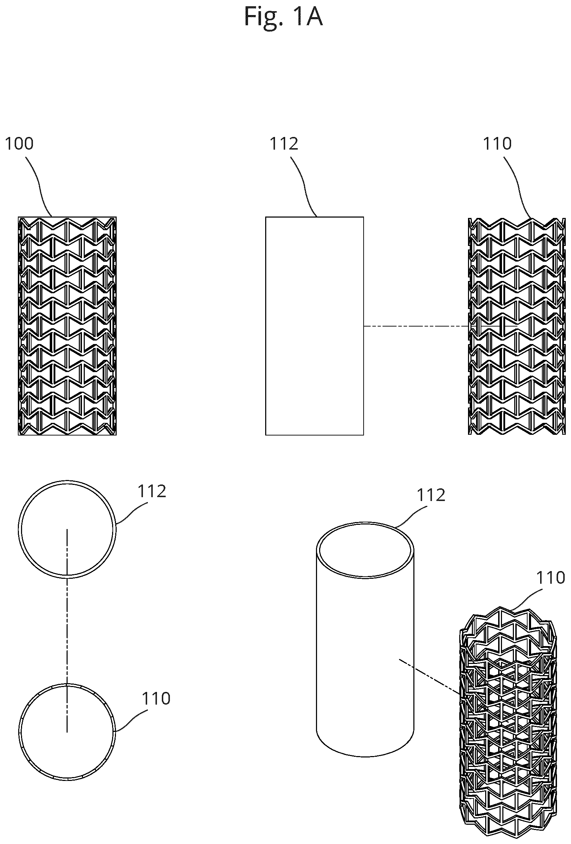

Figure 1A shows a conduit 100 comprising a scaffold 110 and an elastomeric material 112. The scaffold 110 is configured to form a circumferential structural body of the conduit 100 and the elastomeric material 112 is configured to form a fluid impermeable circumferential wall of the conduit 100 conforming to the dimensions of the scaffold 110, ranging from a proximal end of the conduit to a distal end of the conduit, wherein the end of the conduit 100 at which the bodily fluid enters the conduit 100 is identified as the proximal end of the conduit and the end of the conduit 100 at which the bodily fluid exits the conduit 100 is identified as the distal end of the conduit. In this example the elastomeric material 112 has resilient and stretchable properties, wherein the elastomeric material 112 is configured to be compatible with the auxetic properties of the scaffold 110. In an example, the elastomeric material is configured to maintain conformance with deformation of the scaffold. The elastomeric material 112 may comprise, or may be made of, natural or synthetic elastomers, polymers, hydrogels or a combination of these materials. In one example, the elastomeric material 112 is biocompatible.

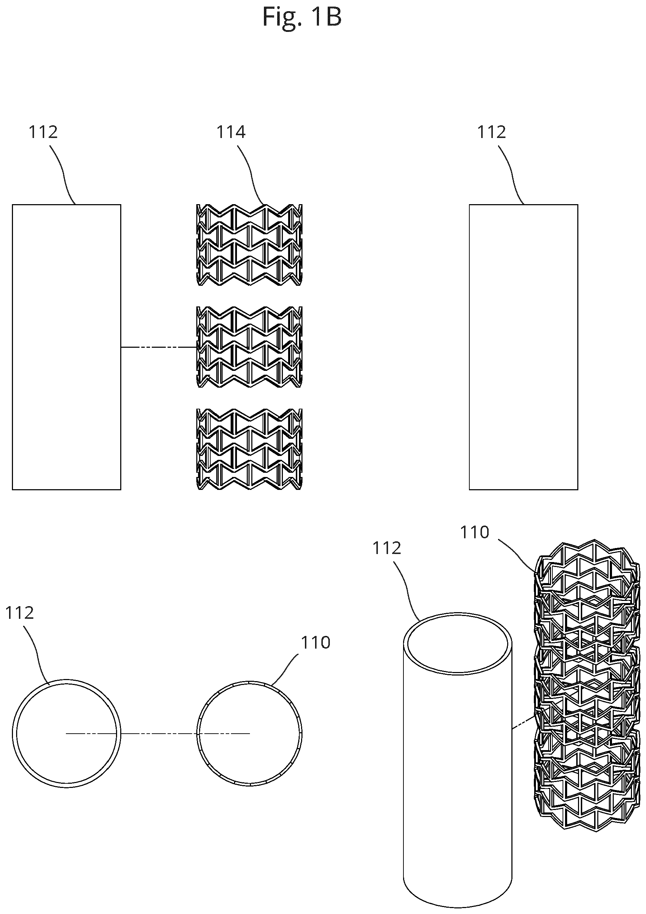

Figure 1B shows the scaffold 110 without the elastomeric material 112. In the example of figure 1B, the scaffold 110 is circumferentially ranging, from a proximal end of the conduit to a distal end of the conduit, wherein the end at which the bodily fluid enters the conduit 100 is identified as the proximal end of the conduit 100 and the end at which the bodily fluid exits the conduit 100, is identified as the distal end of the conduit. The scaffold is configured to provide circumferential structural support to the conduit, from proximal end of the conduit to a distal end of the conduit.

The material and structure of the scaffold 110 is plastically deformable by example means of an external force. The material and structure of the scaffold 110 is deformable, for example in the form of expansion concomitantly in the radial and axial directions, by for example means of an external force, from a first dimension to a first expanded dimensions. The scaffold is configured to remain in its deformed, for example expanded, dimensions. In this example, the scaffold is plastically deformed in the dimensions of the first expanded dimensions of the scaffold. In this way, the scaffold is configured to, for example, remain in the first expanded dimensions and the scaffold does not return back to the first dimensions.

The material and structure of the scaffold may be further deformed by for example means of an external force. After each deformation the scaffold may be plastically deformed, to remain in its specific expanded dimensions and the scaffold may not return back to its previous dimensions. The material and structure of the scaffold can be deformed multiple times. In one example, the material may only be deformable, in the form of expansion, in radial and axial directions.

The scaffold 110 may have auxetic properties due to its macroscopic structure and or microscopic structure. In the example of figure 1A, the scaffold 110 has auxetic properties due to its macroscopic structure. In another example, the scaffold 110 may have auxetic properties due to its microscopic structure. In a yet another example, the scaffold 110 may have auxetic properties due its microscopic and macroscopic structures. In another example, the scaffold 110 may have auxetic properties due a combination of auxetic structures and non-auxetic structures. In one example, the auxetic structures and the non-auxetic structures may be dispersed throughout the scaffold 110 and yet in another example the auxetic structures and the non-auxetic structures alternate each other in a periodic or non-periodic sequence throughout the scaffold 110.

The material of the scaffold 110 may comprise, or may be made of, polymers and elastomers, hydrogels, metal and alloys, carbon-based materials, or a combination of these materials. More specifically, the material of the scaffold may comprise, or may be made of, Co-Cr, surgical steel, nitinol or any other material comprising auxetic properties. In one example, the auxetic material of the scaffold is biocompatible.

In another embodiment, the scaffold 110 is circumferentially ranging from a proximal end of the conduit to a distal end of the conduit, such as scaffold 114, wherein the scaffold 114 is configured to provide structural support over partial segments of the conduit.

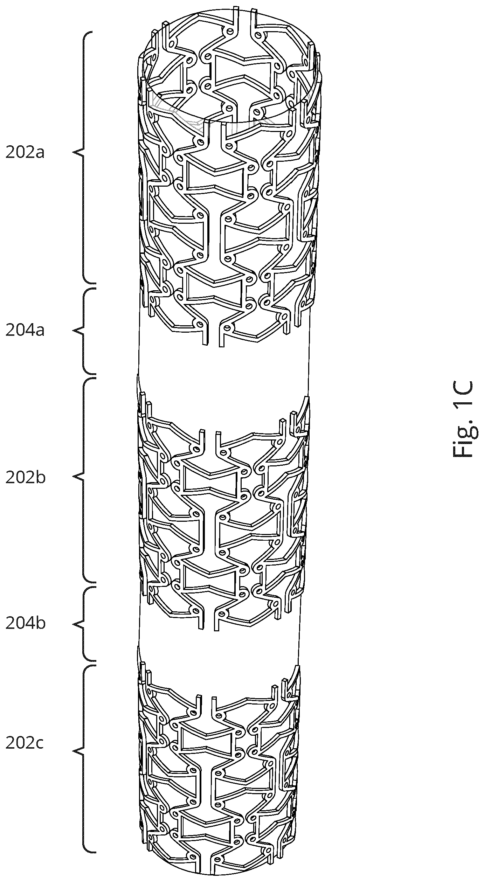

Figure 1C shows an open cell conduit 200, wherein in this example the scaffold 114 is circumferentially ranging from a proximal end of the open cell conduit to a distal end of the open cell conduit, wherein the end at which the bodily fluid enters the open cell conduit is identified as the proximal end of the open cell conduit and the end at which the bodily fluid exits the open cell conduit 200, is identified as the distal end of the open cell conduit. In one example, the scaffold 114 may be segmented at the proximal end, distal end and intermediate part of the open cell conduit 200. In another example, the scaffold 114 may be segmented at the intermediate part of the open cell conduit 200. In yet another example, the scaffold 114 may be segmented at only the proximal end and or the distal end of the open cell conduit 200. In another example, the scaffold 114 may be segmented at a random manner from the proximal end of the open cell conduit to the distal end of the open cell conduit.

In the example of figure 1C, the elastomeric material 112 is configured to continuously form an impermeable circumferential wall of the open cell conduit 200 conforming to the dimensions of the scaffold 114, ranging from a proximal end of the open cell conduit to a distal end of the open cell conduit. The open cell conduit 200 comprises the scaffold 114 and the elastomeric material 112 in the closed segments 202A, 202B, 202C of the open cell conduit 200, wherein the scaffold 114 provides structural support over partial segments of the open cell conduit 200. In one example, in the closed segments 202A, 202B, 202C of the open cell conduit 200, the open cell conduit 200 may be rigid and not bendable.

In another example, in the closed segments 202A, 202B, 202C of the open cell conduit 200, the open cell conduit 200 may be flexible and bendable. In the open segments 204A, 204B of the open cell conduit 200, the open cell conduit 200 comprises the elastomeric material 112 configured to form a fluid impermeable circumferential wall of the open cell conduit 200 without the scaffold 114. In one example, in the open segments 204A, 204B without scaffold 114 of the open cell conduit 200, the conduit 200 may be resilient and bendable. In another example, the open segments 204A, 204B without scaffold 114 of the open cell conduit 200, the conduit 200 may be rigid and not bendable.

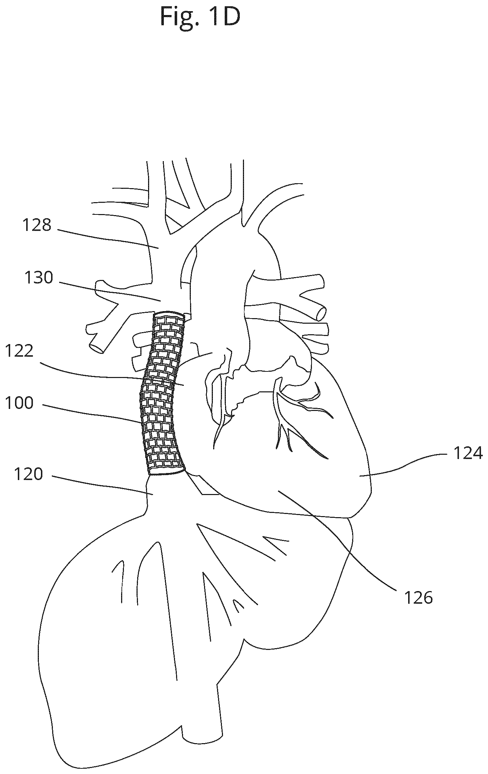

Figure 1D shows a conduit 100 between two vessels of a mammal body in case of a fontan tunnel procedure. In a healthy mammal body, deoxygenated blood from the lower half of the body is transported through the inferior vena 120 cava to the right atrium 122 of the heart 124 into right ventricle 126 of the heart 124. From the upper half of the body deoxygenated blood is transported from the superior vena cava 128 to the right atrium 122 of the heart 124 to the right ventricle 126 of the heart 124. The deoxygenated blood is further transported from the right ventricle 126 to the pulmonary arteries 130 into the lungs for oxygenation of the blood.

In case of a patient with a univentricular heart disease with a fontan tunnel procedure, the deoxygenated blood from the lower half of the body is transported through the inferior vena cava 120 through a conduit 100 to the pulmonary arteries 130 and deoxygenated blood from the upper half of the body is transported from the superior vena cava 128 directly to the pulmonary arteries. From the pulmonary arteries 130 the deoxygenated blood, originating from both the inferior vena cava 120 and the superior vena cava 128, is further transported into the lungs for oxygenation of the blood. In this example, the conduit 100 is fluidly connecting the inferior vena cava 120 with the pulmonary arteries 130 of a mammal body. The proximal end of the conduit 100 is operatively connected to the inferior vena cava 120 from which deoxygenated blood flows from the lower half of the body into the conduit 100 to the distal end of the conduit 100, where the conduit 100 is operatively connected to the pulmonary arteries 130, from which the deoxygenated blood from the lower half an upper half of the body is further transported into the lungs for oxygenation of the blood.

The operative connection between the conduit 100 and the inferior vena cava 120, and the conduit 100 and the pulmonary arteries 130, is fluidly impermeable. The conduit 100 forming the connection between the inferior vena cava 120 and the pulmonary arteries 130 forms a fluidly impermeable layer between an intravascular environment of the mammal body and an extravascular environment of the mammal body. In this example the conduit 100 connects the two vessels in a linear line. In an open cell conduit 200, the open cell conduit 200 may connect the two vessels in a non-linear line because of the open segments of the open cell conduit 100 allowing bending and curvature in the open segments 204 of the open cell conduit 200. In another example, the conduit 100 and the open cell conduit 200 may be used complementary.

In another embodiment, the conduit 100 or the open cell conduit 200 may also be used as a connection unit between the superior vena cava and the pulmonary arteries. In yet another embodiment, the conduit 100 or the open cell conduit 200 may also be used to connect two other vessels at a part of the mammal body, such as in case of kidney diseases, where reconnection of two vessels is required in order to create, recreate or maintain vascular access for haemodialysis.

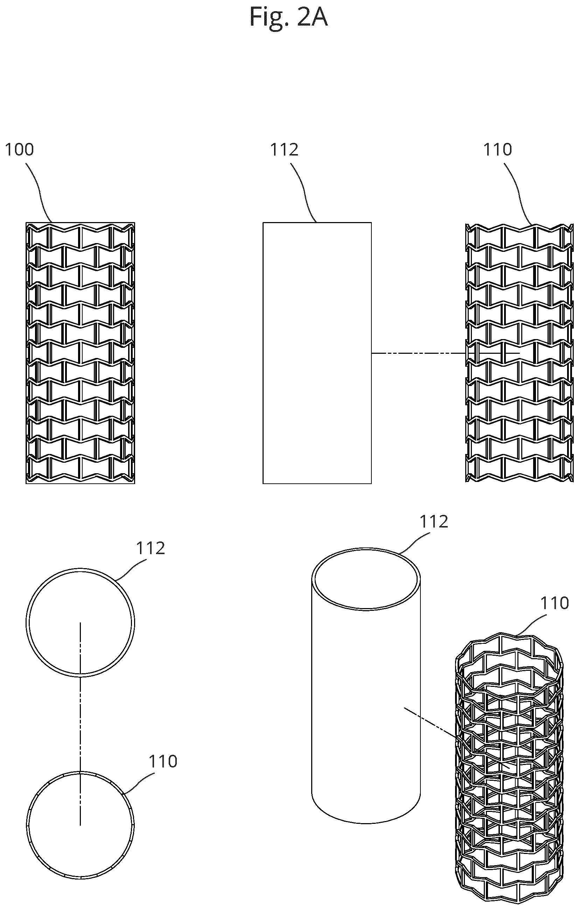

Figure 2A shows the conduit 100 in a first dimension. In this embodiment, the conduit 100 is not expanded, the conduit 100 is compressed axially and radially. In this way, the struts 206, forming the individual auxetic structures of scaffold 110, are in close proximity to each other.

The dimensions of the conduit 100 comprises the length of the conduit, the inner diameter of the conduit, the outer diameter of the conduit, and the circumferential wall thickness of the conduit. The first dimensions of the struts 206 may differ depending on the structure of the struts. The first dimensions of the conduit 100 are patient specific, meaning that the first dimensions of the conduit 100, comprising the inner diameter of the conduit, the outer diameter of the conduit, and the circumferential wall thickness of the conduit, are compatible with the dimensions of the vessels of the patient during implantation, wherein the dimensions of the vessels of the patient comprise the inner diameter of the vessels, the outer diameter of the vessels, and the circumferential wall thickness of the vessels.

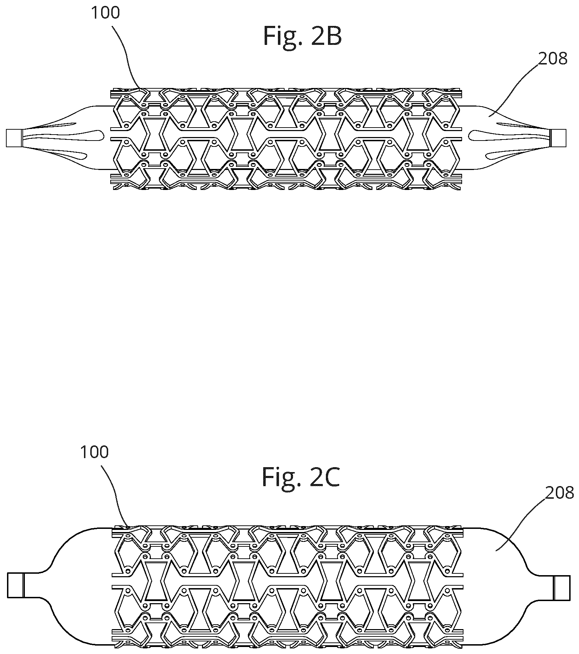

Figure 2B shows a conduit 100 with a balloon 208 that is uninflated, positioned within the internal environment of the conduit 100. In this example, the uninflated balloon 208 is positioned within the internal environment of the conduit 100, extending from a proximal end of the conduit to a distal end of the conduit. In another example, the uninflated balloon 208 is positioned within the internal environment of the conduit 100, extending from a proximal end of the conduit to an intermediate part of the conduit. In yet another example, the uninflated balloon 208 is positioned within the internal environment of the conduit 100, extending from a distal end of the conduit to an intermediate part of the conduit. In another example, the uninflated balloon 208 may be positioned within the internal environment of the conduit 100 in an intermediate segment of the conduit. In yet another example, the uninflated balloon 208 may be positioned within the internal environment of the conduit 100 at a random segment of the conduit. In one example, the uninflated balloon 208 may be positioned at the external environment of the conduit at any random segment of the conduit 100.

The internal environment of the conduit comprises the volume of the conduit through which bodily fluids may flow. The internal environment of the conduit may form the intravascular environment after implantation of the conduit between two vessels. The external environment of the conduit may be situated in the extravascular environment

In the example of figure 2B, the uninflated balloon 208 does not fully occupy the internal environment of the conduit 100 and there is a void between the outer surface of the uninflated balloon and the inner surface of the conduit 100.

The uninflated balloon 208 positioned within the internal environment of the conduit 100 may be inflated by means of an external inflation mechanism. Figure 2C shows an illustration of the conduit 100 with the balloon 208 in an inflated state, wherein the balloon is positioned within the internal environment of the conduit 100. In this example, the inflated balloon 208 is positioned within the internal environment of the conduit 100, extending from a proximal end of the conduit to a distal end of the conduit. In another example, the inflated balloon 208 is positioned within the internal environment of the conduit 100, extending from a proximal end of the conduit to an intermediate part of the conduit. In yet another example, the inflated balloon 208 is positioned within the internal environment of the conduit 100, extending from a distal end of the conduit to an intermediate part of the conduit 100. In another example, the inflated balloon 208 is positioned within the internal environment of the conduit 100 in an intermediate segment of the conduit. In yet another example, the inflated balloon 208 may be positioned within the internal environment of the conduit 100 at a segment of the conduit. In one example, the inflated balloon 208 may be positioned at the external environment of the conduit at any random segment of the conduit 100.

In another embodiment, such as an open cell conduit 200, the conduit 200 may be expanded through segment-by-segment expansion, by means of balloon angioplasty. In this example, the balloon 208 may have dimensions similar or smaller to the dimensions of partial segments of the conduit 100. In this way, the open cell conduit 200 may be expanded partial segment by partial segment. For example, first the partial segment 202A of the conduit 200 may be expanded by means of a balloon, followed by a second partial segment 204A, and subsequently, a third partial segment 202B, a fourth partial 202B, a fifth partial 204B, and a sixth partial 202C of the open cell conduit 200, until all the partial segments of the open cell conduit 200 are expanded and in this way the open cell conduit 200 is expanded from its proximal end to its distal end.

In this example of figure 2C, the balloon 208 is inflated such that the outer surface of the balloon is in close, tight adjacent contact with the inner wall of the conduit. In this way, there is no void between the outer surface of the inflated balloon and the inner wall of the conduit. This may result in pressure applied by the outer surface of the inflated balloon 208 on the inner wall of the conduit 100, resulting in concomitantly expansion of the conduit 100 in radial and axial directions due to the auxetic structure of the scaffold 110 of the conduit 100, in this example, from the proximal end of the conduit to the distal end of the conduit. In the example of figure 2C, the conduit 100 is in its first expanded dimensions, wherein the dimensions of the conduit 100 have changed from the first dimensions to the first expanded dimensions. Further inflation of the balloon 208 further tightness the outer surface of the balloon adjacent to the inner wall of the conduit 100, accordingly more force, in the form of pressure, may be applied to the inner wall of the conduit 100. As a result, the conduit 100 may expand further in dimensions.

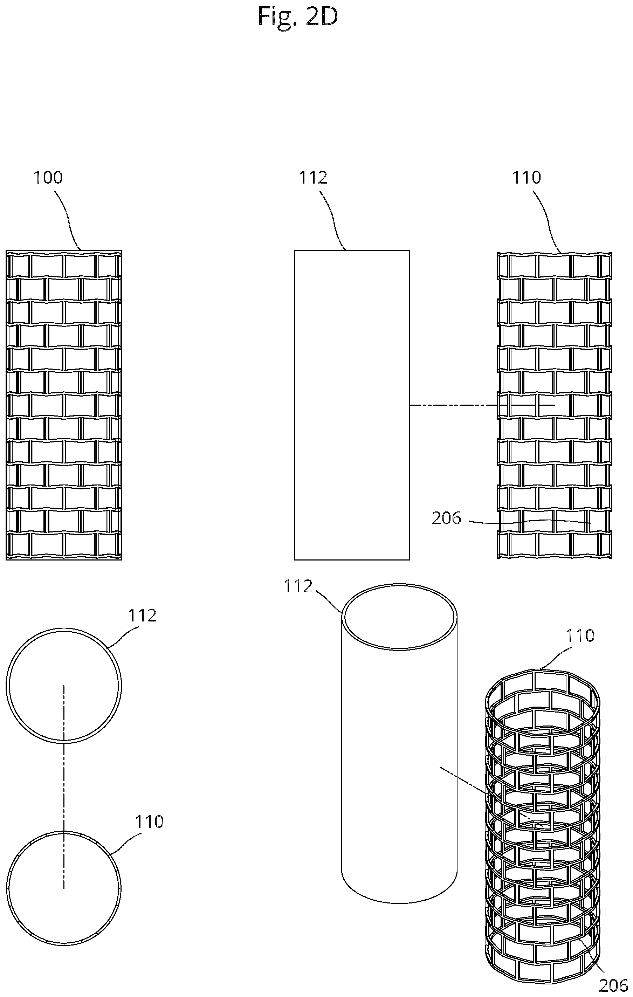

Figure 2D shows a conduit 100 in a first expanded dimensions. In this example, the conduit 100 is expanded concomitantly in radial and axial directions from a first dimensions to a first expanded dimensions, wherein the length of the conduit, the inner diameter of the conduit, the outer diameter of the conduit, and the circumferential wall thickness of the conduit are greater than in the first dimensions of the conduit 100. In this way, the struts 206, forming the individual auxetic structures of scaffold 110, are in a less close proximity to each other than in the first dimensions of the conduit 100. In the first expanded dimensions of conduit, the dimensions of the conduit are compatible with the dimensions of the vessels of the patient after a first somatic growth of the patient, wherein the dimensions of the vessels of the patient comprise the inner diameter of the vessels, the outer diameter of the vessels, and the circumferential wall thickness of the vessels.

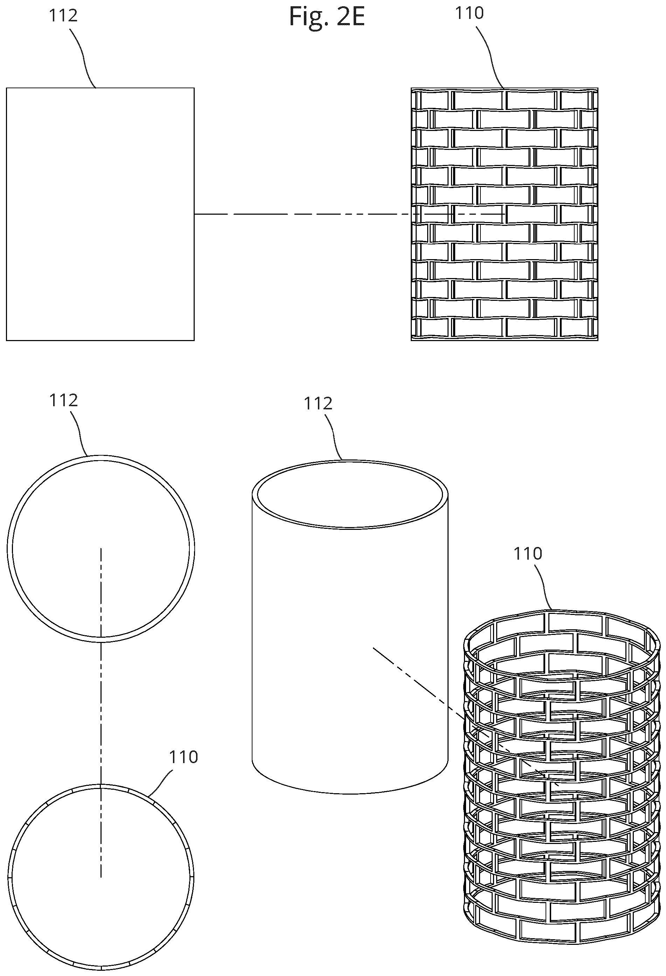

Figure 2E shows a conduit 100 in a next expanded dimension, as a result of further inflation of the inflated balloon by means of an external inflation mechanism. In this example, the length of the conduit, the inner diameter of the conduit, the outer diameter of the conduit, and the circumferential wall thickness of the conduit are greater than in the first expanded dimension of the conduit 100. In this way, the struts 206 are in a further distance relative to each other than in the first expanded dimensions of the conduit 100. In the next expanded dimensions of conduit, the dimensions of conduit are compatible with the dimensions of the vessels of the patient after a next somatic growth of the patient.

Supplementary to the exemplary embodiment provided, the skilled person will appreciate that the conduit may be used to connect two or more other vessels at a part of the mammal body, such as in cases of kidney diseases or other coronary heart diseases, where connection of two vessels by means of a conduit is required to create, recreate or maintain body fluid connection in a mammal body. It will also be understood that different variations are possible within the principles of the conduit and applications described above. The embodiments and implementations are provided as illustrative examples to aid with understanding. The terms "vascular graft", "tunnel", and "conduit" may be used interchangeably.

## Claims

1. A conduit for fluidly connecting two vessels of a mammal body,
the conduit comprising;
a scaffold providing a circumferential support to the conduit;
an elastomeric material provided on the scaffold, configured to form a fluid impermeable circumferential wall of the conduit conforming to the dimensions of the scaffold, ranging from a proximal end of the conduit to a distal end of the conduit;
wherein the conduit is configured such that dimensions of the conduit are adaptable to adjustments in dimensions of the vessels, the adjustments in dimensions of the vessels being driven by a somatic growth of the mammal body.

2. The conduit according to claim 1, wherein the scaffold comprises of auxetic structures.

3. The conduit according to claims 1 and 2, wherein the scaffold is configured to provide circumferential structural support from a proximal end of the conduit to a distal end of conduit.

4. The conduit according to any one of the preceding claims, wherein the scaffold is configured to provide structural support over partial segments of the conduit.

5. The conduit according to claim 4, wherein the conduit comprises multiple scaffold partial segments, connected by the fluid impermeable circumferential wall of the conduit.

6. The conduit according to any one of the preceding claims, wherein the scaffold has an open cell design.

7. The conduit according to any one of the preceding claims, wherein the open cell design of the scaffold is configured to provide natural flexibility to the conduit.

8. The conduit according to any one of the preceding claims, wherein the elastomeric material and auxetic material are biocompatible.

9. The conduit according to any one of the preceding claims, wherein
the auxetic material may comprise:
Co-Cr
surgical steel
nitinol
polymers and elastomers
hydrogels
metal and alloys
carbon-based materials

10. The conduit according to any one of the preceding claims, wherein the elastomeric material is configured to be compatible with the auxetic properties of the scaffold.

11. The conduit according to any one of the preceding claims, wherein the scaffold and elastomeric material are configured to form the conduit.

12. The conduit according to any one of the preceding claims, wherein the conduit and the scaffold in particular is configured to be expanded concomitantly in radial and axial directions.

13. The conduit according to any one of the preceding claims, wherein the conduit is configured to be expanded from first dimensions to first expanded dimensions.

14. The conduit according to any one of the preceding claims, wherein the conduit is configured to be expanded from a first expanded dimension to next expanded dimension.

15. The conduit according to any one of the preceding claims, wherein the conduit is configured to be expanded multiple times to further increment the dimensions of the conduit.

16. The conduit according to any one of the preceding claims, wherein the conduit is configured to remain in its expanded dimension.

17. The conduit according to any one of the preceding claims, wherein the conduit is configured to be further expanded from its expanded dimension.

18. The conduit according to any one of the preceding claims, wherein the conduit and the scaffold in particular is configured to be expanded by means of an external mechanical force.

19. The conduit according to claim 18, wherein the external mechanical force is applied by means of balloon angioplasty.

20. The conduit according to claim 6-7 and 12-19, wherein the open cell design is configured to allow segment-by-segment expansion of the conduit.

21. The conduit according to any one of the preceding claims, wherein the first dimensions of the conduit are compatible to the dimensions of the vessels during implantation, and the next dimensions of the conduit are compatible to the dimensions of vessels after a next growth of the vessels.

22. Conduit according to the preceding claims, wherein the conduit is configured to replace a portion of a vessel.

23. Conduit according to the preceding claims, wherein the conduit is configured to form a fluid impermeable layer between an intravascular environment of the mammalian body and an extravascular environment of the mammalian body.

24. Conduit according to the preceding claims, wherein the conduit is configured to facilitate the transportation of bodily fluids.

25. Conduit according to the preceding claims, wherein the conduit is coated to promote adhesions of desired cells and inhibit the adhesion of undesired cells.

26. Conduit according to the preceding claims, wherein the conduit is patient specific.
